# EUROPEAN PATENT SPECIFICATION

(11) **EP 2 921 473 B1**
(45) Date of publication and mention of the grant of the patent: **27.09.2017**
(21) Application number: 13845848.4
(22) Date of filing: 08.10.2013
(51) Int. Cl.: C07C 255/38, C07C 253/30, C07C 217/60, C07C 213/02, C07C 233/18, C07C 231/02

(54) **1-CYAN-1-(7-METHOXYL-1-NAPHTYL) METHANOL ESTER COMPOUND AND PREPARATION METHOD AND USE THEREOF**
1-CYAN-1-(7-METHOXYL-1-NAPHTYL)-METHANOLESTERVERBINDUNG SOWIE HERSTELLUNGSVERFAHREN UND VERWENDUNG DAVON
COMPOSÉ 1-CYANO-1-(7-MÉTHOXY-1-NAPHTYL) MÉTHANOL ESTER ET PROCÉDÉ DE PRÉPARATION ET UTILISATION CORRESPONDANTS

(30) Priority: 09.10.2012 CN 201210378968; 09.10.2012 CN 201210378868
(43) Date of publication of application: 23.09.2015
(73) Proprietor: Jiangxi Synergy Pharmaceutical Co. Ltd., Jiangxi 330700 (CN)
(72) Inventor: JIANG, Yuansen, Yichun Jiangxi 330700 (CN); YIN, Qinghua, Yichun Jiangxi 330700 (CN); XU, Hongcai, Yichun Jiangxi 330700 (CN); JIANG, Huigang, Yichun Jiangxi 330700 (CN)
(74) Representative: Schiweck, Weinzierl & Koch Patentanwälte Partnerschaft mbB
(86) International application number: PCT/CN2013/084836
(87) International publication number: WO 2014/056421

(56) References cited:
- EP-A1- 1 564 202
- WO-A2-2012/046253
- CN-A- 1 680 284
- CN-A- 101 096 346
- CN-A- 102 276 492
- CN-A- 102 875 408
- US-A- 5 194 614
- Kindler ET AL: "Studien iiber den Mechanismus cheinischer Reaktionen XI. Uber die Lenkung der katalytischen Hydrierung bei Estern des Mandelsäurenitrils", Justus Liebigs Annalen der Chemie, 1 January 1931 (1931-01-01), pages 49-54, XP055270651, Retrieved from the Internet: URL:http://onlinelibrary.wiley.com/store/1 0.1002/jlac.19495640108/asset/19495640108_ ftp.pdf?v=1&t=inssdbsq&s=8fa5da66c5d2dccb3 1d07606dc939e4b400e3e91 [retrieved on 2016-05-04]

## Description

### TECHNICAL FIELD

The invention belongs to the field of organic chemistry, specifically relates to a new 1-cyano-1-(7-methoxyl-1-naphthyl) methyl ester compound and preparation method and use thereof.

### BACKGROUND

Agomelatine (N-[2-(7-methoxyl-1-naphthyl)ethyl] acetamide), represented by structural formula III, is the first antidepressant of melatonin receptor agonist in the world, its unique acting targets can make sleep quality of patients improved at the same time of treating depression.

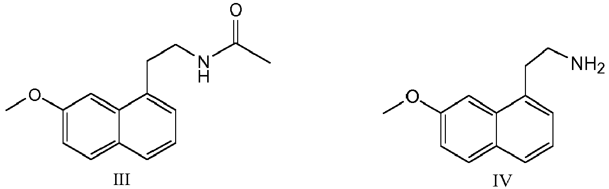

The key intermediate of agomelatine: 2-(7-methoxyl-1-naphthyl) ethylamine of formula IV, which can be acylated by acetic anhydride to obtain agomelatine. The preparation methods of 2-(7-methoxyl-1-naphthyl) ethylamine have been reported in a number of literatures. The common synthetic routes are as follows:

### Route I: (Specification of Chinese Patent for Invention No. CN101709036B)

Tianjin Institute of Pharmaceutical Research used 2-(7-methoxyl-1-naphthyl) acetamide of structural formula V as a starting material, in the presence of organic solvents, reducing agents and Lewis acid, to obtain 2-(7-methoxyl-1-naphthyl) ethylamine of formula IV and the hydrochloride thereof by reduction via a one-step reaction.

The patented method has the following disadvantages:
(1) Moisture is extremely restricted, because the Lewis acid used, i.e. boron trifluoride ethyl ether, hydrolyzes immediately when exposed to moisture in the air and breaks down to highly toxic hydrogen fluoride fume, which is adverse to safe operation.
(2) The yield is not high, only 72.2%; and the price of various raw materials for the reaction is high, which are not suitable for industrial production.

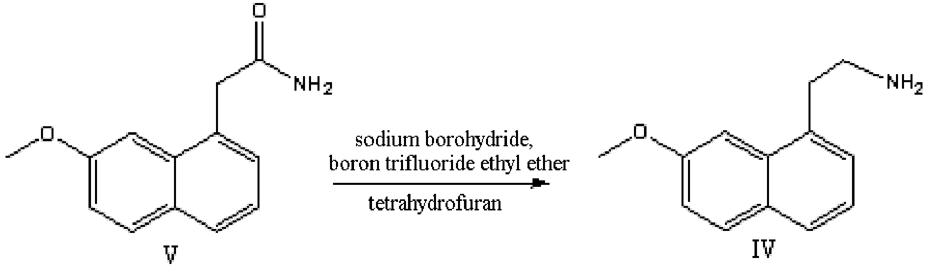

### Route II: (Specifications of Chinese Patent for InventionNo. CN1321106C and European Patent Application No. EP0447285)

Les Laboratoires Servier in France disclosed a preparation method of agomelatineby, in which 2-(7-methoxyl-1-naphthyl) acetonitrile of formula VI is catalyzed by Raney nickel, and subjected to hydrogenation reduction under high pressure in the presence of ammonia water and ethanol to obtain 2-(7-methoxyl-1-naphthyl) ethylamine of formula IV.

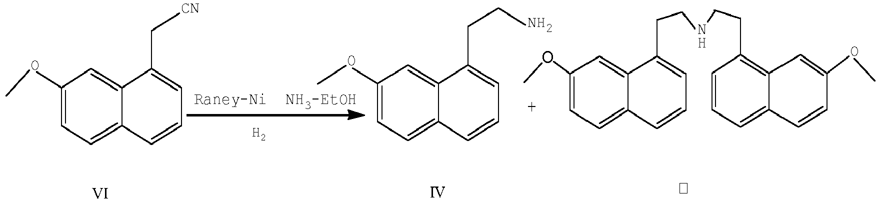

The defects of the method lie in: hydrogenation reduction must be conducted under high pressure (3 Mpa), resulting in high requirement for production equipment, so it is not suitable for industrialization-scale production; additionally, it concomitantly generates coupling by-products of formula VII whose properties are similar to 2-(7-methoxyl-1- naphthyl) ethylamine , which makes it very difficult to perform purification.

### SUMMARY OF THE INVENTION

Accordingly, against the deficiencies of the prior art the present invention provides a novel compound, which can be catalyzed by Raney nickel under mild conditions to obtain the key intermediate of agomelatine, i.e. 2-(7-methoxyl-1-naphthyl) ethylamine, wherein the reaction produces less by-products, easy for purification. With the application of the new compound, it also brings a new synthetic method of agomelatine. This synthetic method has advantages of short synthetic route, simple reaction conditions, cheap and abundant raw materials, mild reaction conditions, environment friendly, high product yield and high purity and so on, so it is especially suitable for large-scale industrial production.

To achieve the above objects, the present invention adopts the following technical solutions:
A compound of formula I, 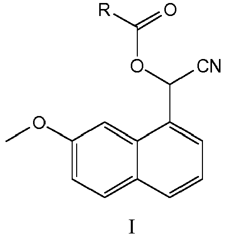 wherein R is methyl, ethyl, propyl.

An object of the present invention is to provide a method for preparing the compound of formula I: a compound of formula II reacts with organic acid to obtain the compound of formula I in the presence of aromatization reagent.

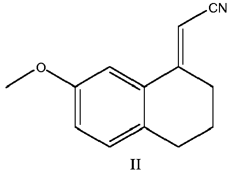

Preferably, the mole ratio of the aromatization reagent to the compound of formula II is 1-10:1; more preferably, the mole ratio of the aromatization reagent to the compound of formula II is 2-2.5:1.

Preferably, the aromatization reagent is 2,3-dichloro-5,6-dicyano-1,4-benzoquinone (DDQ) or tetrachlorobenzoquinone; more preferably, the aromatization reagent is tetrachlorobenzoquinone.

Preferably, the organic acid is selected from any one of acetic acid, propanoic acid and n-butyric acid.

Another object of the present invention is to provide a use of the compound of formula I in the preparation of the key intermediate of agomelatine i.e. 2-(7-methoxyl-1-naphthyl) ethylamine as represented by formula IV: the compound of formula I in an ammoniacal C₁-C₄ fatty alcohol is subjected to hydrogenation reduction under catalyzation by Raney nickel to obtain the 2-(7-methoxyl-1-naphthyl) ethylamine. 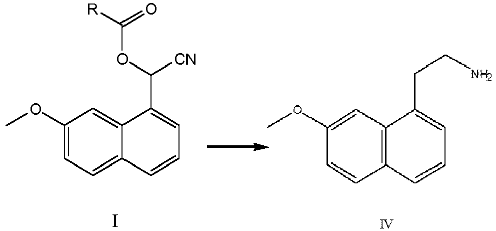 wherein R is methyl, ethyl or propyl.

Preferably, the pressure for the hydrogenation reduction reaction is 0.1-2 Mpa; more preferably, the pressure for the hydrogenation reduction reaction is 0.3-0.8 Mpa.

The C₁-C₄ fatty alcohol is selected from methanol, ethanol, propanol, isopropanol, n-butanol, isobutanol or tertiary butanol; more preferably, the C₁-C₄ fatty alcohol is ethanol.

Yet another object of the present invention is to provide a synthetic method of agomelatine, comprising that a compound of formula I in an ammoniacal C₁-C₄ fatty alcohol is subjected to hydrogenation reduction under catalyzation by Raney nickel to prepare 2-(7-methoxyl-1-naphthyl) ethylamine, 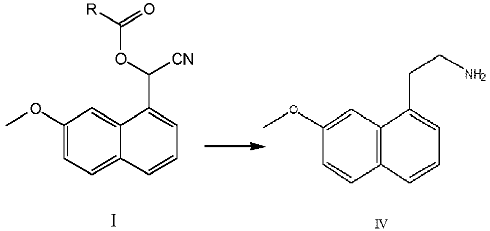 wherein R is methyl, ethyl or propyl.

Preferably, the pressure for the hydrogenation reduction reaction is 0.1-2 Mpa; more preferably is 0.3-0.8 Mpa.

Preferably, the C₁-C₄ fatty alcohol is selected from methanol, ethanol, propanol, isopropanol, n-butanol, isobutanol or tertiary butanol; more preferably is ethanol.

The synthetic method of agomelatine of the present invention also comprises that 7-methoxyl-1-tetralone of formula VIII, 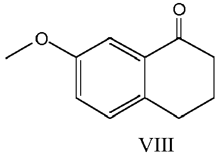 under catalyzation by sodium hydride, reacts with dimethyl cyanomethylphosphonate in an aprotic polar solvent to obtain (7-methoxyl-3,4-dihydro-2H-naphth-1-ylidene) acetonitrile of formula II,

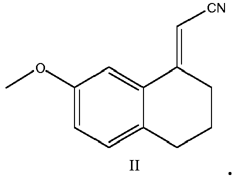

Preferably, the aprotic polar solvent is N,N-dimethylformamide, N,N-dimethylacetamide, ethylene glycol dimethyl ether, dimethyl sulfoxide or sulfolane; more preferably is ethylene glycol dimethyl ether.

The synthetic method of agomelatine of the present invention further comprises that 2-(7-methoxyl-1-naphthyl) ethylamine reacts with acetic anhydride to generate agomelatine which is then separated in solid form.

As a preferred embodiment of the present invention, a method for preparing agomelatine specifically comprises the following steps:
1) Charging ethylene glycol dimethyl ether and sodium hydride into a reaction vessel, controlling the temperature at 15±2 °C, adding the compound of formula (VIII), adding dimethyl cyanomethylphosphonate dropwise within 3-4 hours; controlling the temperature at 10 °C and stirring the mixture for 15 hours; cooling the mixture to a temperature below 5 °C, then slowly adding a small amount of water to deplete the remaining sodium hydride; and then adding 10% hydrochloric acid solution dropwise to adjust the pH to 5-6; adding dichloromethane for extraction, and concentrating the organic layer under reduced pressure until dry; adding ethanol into the residue for dissolution by heating, and then cooling the resulting solution for crystallization, followed by filtration to give (7-methoxyl-3,4-dihydro-2H-naphth-1-ylidene) acetonitrile;
2) Charging tetrachlorobenzoquinone, glacial acetic acid and (7-methoxyl-3,4-dihydro-2H-naphth-1-ylidene) acetonitrile prepared in step 1 into a reaction vessel followed by stirring; heating the mixture to refluxing, and allowing for reaction for 12 hours; cooling the reaction solution to room temperature, then distilling the reaction solution under reduced pressure; dissolving the residue by adding toluene followed by filtration; washing the filtrate with 5% sodium sulfite solution and then with water, followed by filtration, allowing for layering; concentrating the toluene layer under reduced pressure, dissolving the residue by adding ethanol followed by stirring, cooling the mixture for crystallization, followed by filtration to give 1-cyano-1-(7-methoxyl-1-naphthyl) methyl acetate;
3) Charging 1-cyano-1-(7-methoxyl-1-naphthyl) methyl acetate prepared in step 2, Raney nickel, ethanol and ammonia water into an autoclave which is then purged with nitrogen, introducing hydrogen gas; controling the pressure at 0.3-0.8 Mpa and the temperature at 40-60 °C, allowing for reaction for 7 hours; cooling the reaction solution followed by filtration to recover Raney nickel; concentrating the filtrate under reduced pressure until dry, followed by washing by adding toluene and water, allowing for layering; and then washing the toluene layer by adding saturated brine, allowing for layering; concentrating the toluene layer under reduced pressure to obtain 1-(7-methoxyl-1-naphthyl) ethylamine;
4) Charging toluene, triethylamine and 2-(7-methoxyl-1-naphthyl) ethylamine prepared in step 3 into a reaction vessel, adding acetic anhydride dropwise slowly at a temperature below 30 °C; upon completion, allowing for reaction for 5 hours at 20-30 °C; after the completion of the reaction, adding water dropwise to hydrolyze the remaining acetic anhydride followed by filtration; washing the filter cake with water to pH=7, washing the toluene layer with water to pH=7, and then pooling the filter cake and the toluene layer of the filtrate, followed by concentration under reduced pressure until dry; adding toluene into the residue, heating for refluxing until the solution turns clear; cooling the solution to 0-5 °C to allow for crystallization, followed by filtration; drying the filter cake to give the agomelatine.

The reaction route involved in the present invention is as follows: 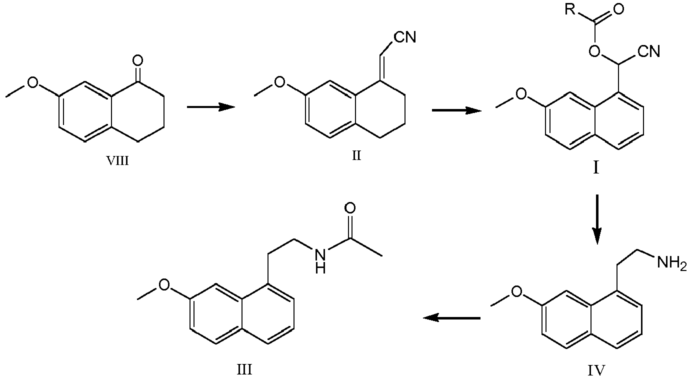 2-(7-methoxyl-1-naphthyl) ethylamine of formula IV can be obtained from the compound of formula I of the present invention via a one-step reaction. The yield of the reaction is high, and the average yield is more than 95%. The reaction is mild, requiring lower pressure, that is, the reaction can be conducted at 0.3-0.8 Mpa; the products of the reaction are relatively simple, comprising no coupling by-product of formula VII, and therefore the post-processing is simple and the yield is high. The compound of formula I of the present invention can be applied to synthesis of agomelatine, resulting in high purity (>99.5%) of the final product agomelatine, which is suitable for large-scale industrial production.

### BRIEF DESCRIPTION OF THE DRAWINGS

Hereinafter, the embodiments of the invention will be described in details with reference to the accompanying drawings, wherein:
Figure 1 is a HPLC chromatogram of the agomelatine synthesized in Example 8, wherein Peak 1 is the absorption peak of agomelatine.
Figure 2 is a HPLC chromatogram of the agomelatine synthesized in Example 9, wherein Peak 1 is the absorption peak of agomelatine.
Figure 3 is a HPLC chromatogram of the agomelatine synthesized in Example 10, wherein Peak 1 is the absorption peak of agomelatine.

### DESCRIPTION OF THE PREFERRED EMBODIMENTS

Hereinafter, the present invention will be further described in details in combination with specific embodiments, and the examples set forth are only for illustrating the present invention but not intended to limit the scope of the invention.

### Example 1: Preparation of (7-methoxyl-3,4-dihydro-2H-naphth-1-ylidene) acetonitrile

200 ml of ethylene glycol dimethyl ether and 17.0 g (0.71 mol) of sodium hydride were charged into a 1,000 ml four-neck flask; while the temperature was controlled at 15±2 °C, 50 g (0.28 mol) of 7-methoxyl-1-tetralone was added, followed by stirring for 30 minutes; 70.4 g (0.40 mol) of diethyl cyanomethylphosphonate was added into the reaction solution dropwise at 15 °C within 3-4 hours; the temperature was controlled at 10 °C, allowing for reaction for 15 hours while stirring; when the reaction was detected to be completed, the reaction solution was cooled to a temperature below 5 °C; 200 ml of water was added dropwise slowly within 30 minutes, the pH value was adjusted to 5-6 with hydrochloric acid; 2×100 ml of dichloromethane was added for extraction twice; the organic layers were concentrated under reduced pressure until dry, 200 ml of 95% ethanol was added into the residue for dissolution by heating , followed by stirring for 1 hour; the resulting solution was then cooled to 0 °C for crystallization while stirring for 2 hours; the crystals were filtrated, followed by blast drying at 40 °C to obtain 56.3 g of the target product, with a yield of 89% and a purity of 99.1 %.

### Example 2: Preparation of 1-cyano-1-(7-methoxyl-1- naphthyl) methyl acetate

85.30 g (0.35 mol) of tetrachlorobenzoquinone and 600 ml of glacial acetic acid were charged into a reaction flask followed by stirring; 30 g (0.15 mol) of (7-methoxyl-3,4-dihydro-2H-naphth-1-ylidene) acetonitrile prepared in Example 1 was added followed by stirring for 1 hour; the mixture was heated to refluxing, allowing for reaction for 12 hours. The reaction solution was cooled to room temperature, and then was distilled under reduced pressure to recover acetic acid; 200 ml of toluene was added into the residue, followed by stirring for 1 hour and then filtration; the filtrate was washed twice with 2×200 ml of 5% sodium bisulfite aqueous solution, then twice with 2×100 ml of water, followed by filtration; the toluene layer was concentrated under reduced pressure; 200 ml of ethanol was added into the residue, followed by stirring at room temperature for 1 hour; the solution was then cooled to 0 °C, followed by stirring for 1 hour, filtration, vacuum drying at 40 °C to obtain 36.9 g of the target compound, with a yield of 90% and a GC purity of 99.9%.
¹H-NMR (400 MHz, CDCl₃) δ2.16 (s, 3H), 4.39 (s, 3H), 7.02 (s, 1H), 7.20-7.23 (m, 1H), 7.27-7.28(d, J=4.0 Hz, 1H), 7.33-7.37 (t, J=8.0 Hz, 1H), 7.75-7.80 (m, 2H), 7.85-7.87 (d, J=8.0, 1H).
¹³C-NMR (400 MHz, CDCl₃) δ20.45, 55.47, 61.50, 101.43, 116.20, 119.27, 122.85, 125.71, 128.33, 129.42, 130.71, 131.23, 131.51, 158.87, 169.14.
MS (ESI): 278[M+Na]⁺.

### Example 3: Use of 1-cyano-1-(7-methoxyl-1-naphthyl) methyl acetate

30.0 g (0.118 mol) of 1-cyano-1-(7-methoxyl-1-naphthyl) methyl acetate prepared in Example 2, 6 g of Raney nickel, 40 ml of 20% ammonia water and 180 ml of 95% ethanol were charged into an autoclave which is then purged with nitrogen, followed by introducing hydrogen gas; the pressure was controlled at 0.4-0.5 Mpa and the temperature was controlled at 40-50 °C; the reaction was stopped after 7 hours; the reaction solution was cooled to room temperature, then Raney nickel was recovered by filtration; the filtrate was concentrated under reduced pressure until dry; followed by washing once by adding 200 ml of toluene was added, followed by washing once with 100 ml of water; allowing for layering; the toluene layer was washed once by adding 100 ml of saturated brine, followed by layering; the toluene layer was concentrated under reduced pressureto obtain 23.26 g of oily substance 2-(7-methoxyl-1-naphthyl) ethylamine with a yield of 98%.
¹H-NMR (400 MHz, CDCl₃) δ1.80 (s, 3H), 3.05-3.08 (t, J=6.0 H_{Z}, 2H), 3.12-3.16 (t, J=8.0 H_{Z}, 2H), 7.14-7.16 (d, J=8.0 H_{Z}, 1H), 7.22-7.31 (m, 3H), 7.63-7.65 (d, J=8.0 H_{Z}, 1H), 7.72-7.74 (d, J=8.0 H_{Z}, 1H).

### Example 4: Preparation of 1-cyano-(7-methoxyl-1-naphthyl) methyl propionate

185 g (0.75 mol) of tetrachlorobenzoquinone and 600 ml of propanoic acid were added into a reaction flask followed by stirring; 30 g (0.15 mol) of (7-methoxyl-3,4-dihydro-2H-naphth-1-ylidene) acetonitrile prepared according to the method described in Example 1 was added followed by stirring for 1 hour; the mixture was heated to refluxing, allowing for reaction for 12 hours. The reaction solution was cooled to room temperature, and then was distilled under reduced pressure to recover propanoic acid; 200 ml of toluene was added into the residue, followed by stirring for 1 hour and then filtration; the filtrate was washed twice with 2×200 ml of 5% sodium bisulfite aqueous solution, then twice with 2×100 ml of water, followed by filtration; the toluene layer was concentrated under reduced pressure; 200 ml of ethanol was added into the residue followed by stirring at room temperature for 1 hour; the solution was then cooled to 0 °C, followed by stirring for 1 hour, filtration, vacuum drying at 40 °C to obtain 36.9 g of the target compound, with a yield of 91% and a GC purity of 99.5%.
¹H-NMR (400 MHz, CDCl₃) δ1.16-1.18 (t, 3H), 2.01-2.04 (m, 2H), 4.38 (s, 3H), 7.03(s, 1H), 7.21-7.24 (m, 1H), 7.27-7.28 (d, J=4.0 Hz, 1H), 7.32-7.36 (t, J=8.0 Hz, 1H), 7.73-7.78 (m, 2H), 7.86-7.88 (d, J=8.0, 1H).
¹³C-NMR (400 MHz, CDCl₃) δ11.2, 27.5, 57.47, 66.3, 102.43, 117.20, 120.27, 124.85, 127.71, 131.33, 131.72, 132.41, 133.56, 135.1, 159.76, 173.14.
MS (ESI): 292[M+Na]⁺.

### Example 5: Use of 1-cyano-(7-methoxyl-1-naphthyl) methyl propionate

31.7 g (0.118 mol) of 1-cyano-(7-methoxyl-1-naphthyl) methyl propionate prepared in Example 4, 6 g of Raney nickel, 40 ml of 20% ammonia water and 180 ml of 95% ethanol were charged into an autoclave which is then purged with nitrogen, followed by introducing hydrogen gas; the pressure was controlled at 0.7-0.8 Mpa and the temperature was controlled at 50-55 °C; the reaction was stopped after 7 hours; the reaction solution was cooled to room temperature, then Raney nickel was recovered by filtration; the filtrate was concentrated under reduced pressure until dry; 200 ml of toluene was added, followed by washing once with 100 ml of water, allowing for layering; the toluene layer was washed once by adding 100 ml of saturated brine, followed by layering; the toluene layer was concentrated under reduced pressure to obtain 22.94 g of oily substance (7-methoxyl-1-naphthyl) ethylamine with a yield of 97%.
¹H-NMR (400 MHz, CDCl₃) δ1.80 (s, 3H), 3.05-3.08 (t, J=6.0 H_{Z}, 2H), 3.12-3.16 (t, J=8.0 H_{Z}, 2H), 7.14-7.16 (d, J=8.0 H_{Z}, 1H), 7.22-7.31 (m, 3H), 7.63-7.65 (d, J=8.0 H_{Z}, 1H), 7.72-7.74 (d, J=8.0 H_{Z}, 1H).

### Example 6: Preparation of 1-cyano-1-(7-methoxyl-1-naphthyl) methyl butyrate

272 g of 2,3-dichloro-5,6-dicyano-1,4-benzoquinone (DDQ) and 700 ml of n-butyric acid were charged into a reaction flask, followed by stirring; 30 g (0.15 mol) of (7-methoxyl-3,4-dihydro-2H-naphth-1-ylidene) acetonitrile prepared according to the method described in Example 1 was added followed by stirring for 1 hour; the mixture was heated to refluxing, allowing for reaction for 12 hours. The reaction solution was cooled to room temperature, and then was distilled under reduced pressure to recover n-butyric acid; 200 ml of toluene was added into the residue, followed by stirring for 1 hour and then filtration; the filtrate was washed twice with 2×200 ml of 5% sodium bisulfite aqueous solution, then twice with 2×100 ml of water, followed by filtration; the toluene layer was concentrated under reduced pressure; 200 ml of ethanol was added into the residue, followed by stirring at room temperature for 1 hour; the solution was then cooled to 0 °C, followed by stirring for 1 hour, filtration, vacuum drying at 40 °C to obtain 38.4 g of the target compound, with a yield of 90% and a GC purity of 99.7%.
¹H-NMR (400 MHz, CDCl₃) δ0.98-1.01 (t, 3H), 1.74-1.76 (m, 2H), 2.24-2.26 (m, 2H), 4.39 (s, 3H), 7.02 (s, 1H), 7.21-7.24 (m, 1H), 7.26-7.27 (d, J=4.0 Hz, 1H), 7.33-7.37 (t, J=8.0 Hz, 1H), 7.77-7.82 (m, 2H), 7.84-7.86 (d, J=8.0, 1H).
¹³C-NMR (400 MHz, CDCl₃) δ15.32, 20.45, 37.58, 55.47, 67.86, 102.43, 116.20, 120.27, 123.13, 127.52, 130.45, 131.47, 132.74, 133.83, 135.45, 157.87, 173.22.
MS (ESI): 306[M+Na]⁺.

### Example 7: Use of 1-cyano-1-(7-methoxyl-1-naphthyl) methyl butyrate

33.4 g (0.118 mol) of 1-cyano-1-(7-methoxyl-1-naphthyl) methyl butyrate prepared in Example 6, 6 g of Raney nickel, 40 ml of 20% ammonia water and 180 ml of 95% ethanol were charged into an autoclave which is then purged with nitrogen, followed by introducing hydrogen gas; the pressure was controlled at 0.5-0.6 Mpa and the temperature was controlled at 40-45 °C; the reaction was stopped after 8 hours; the reaction solution was cooled to room temperature, then Raney nickel was recovered by filtration; the filtrate was concentrated under reduced pressure until dry; 200 ml of toluene was added , followed by washing once with 100 ml of water, allowing for layering; the toluene layer was washed once by adding 100 ml of saturated brine, followed by layering; the toluene layer was concentrated under reduced pressure to obtained 22.7 g of oily substance 2-(7-methoxyl-1- naphthyl) ethylamine with a yield of 96%.
¹H-NMR (400 MHz, CDCl₃) δ1.80 (s, 3H), 3.05-3.08 (t, J=6.0 H_{Z}, 2H), 3.12-3.16 (t, J=8.0 H_{Z}, 2H), 7.14-7.16 (d, J=8.0 H_{Z}, 1H), 7.22-7.31 (m, 3H), 7.63-7.65 (d, J=8.0 H_{Z}, 1H), 7.72-7.74 (d, J=8.0 H_{Z}, 1H).

### Example 8: Synthesis of agomelatine

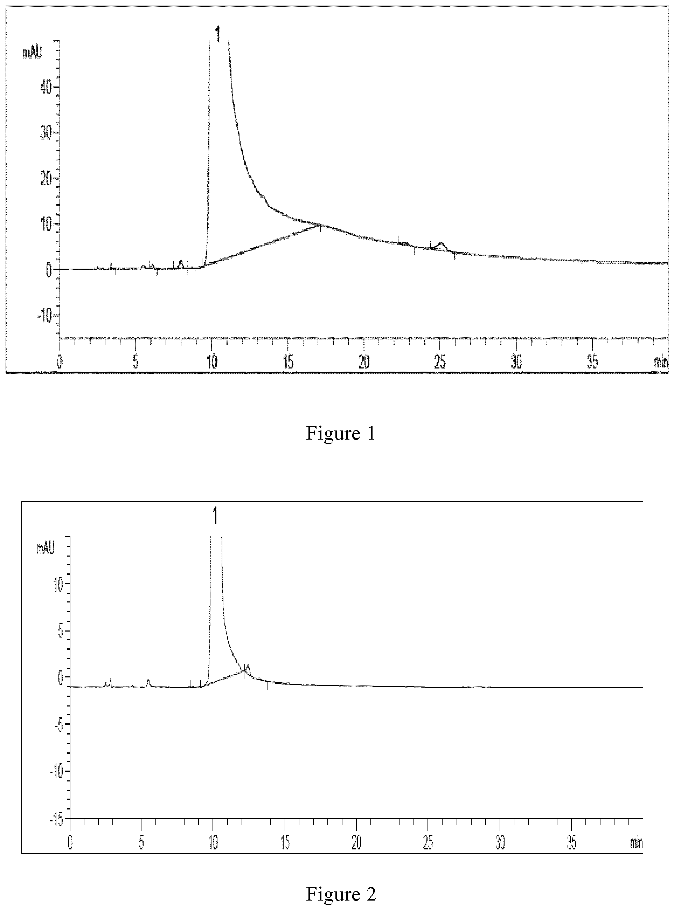

23.26 g of 2-(7-methoxyl-1-naphthyl) ethylamine prepared in Example 3, 20 ml of triethylamine and 120 ml of toluene into a reaction flask, followed by stirring and then cooling to 20 °C; 17 g of acetic anhydride was added dropwise slowly, and the temperature was controlled below 30 °C until addition is completed; the reaction was conducted for 4-5 hours at the temperature of 20-30 °C, when the reaction was detected to reach completion, 100 ml water was added followed by stirring for 10 minutes and then filtration; the filter cake was rinsed with 3×100 ml of water for 3 times, and the filtrate was allowed for layering, where the toluene layer was extracted for 3 times by adding 3×100 ml of water ; the toluene layer and the filter cake was pooled and concentrated under reduced pressure until dry; 100 ml of toluene was added to the residue, followed by heating to refluxing and maintaining the temperature for 30 minutes; the solution was cooled to 0-5 °C to allow for crystallization while stirring for 2 hours, followed by filtration and drying to obtain 25.30 g of white crystalline agomelatine. The yield was 90% and the purity was 99.9%. The HPLC chromatogram was shown in Figure 1.

### Example 9: Synthesis of agomelatine

22.94 g of 2-(7-methoxyl-1-naphthyl) ethylamine prepared in Example 5 was charged into a reaction flask; by following the same operation steps as Example 8, 24.74 g of white crystalline agomelatine was obtained. The yield was 88% and the purity was 99.7%. The HPLC chromatogram was shown in Figure 2.

### Example 10: Synthesis of agomelatine

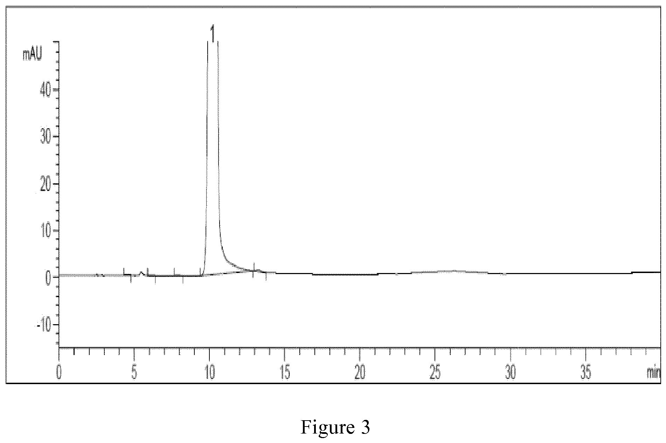

22.7 g of 2-(7-methoxyl-1-naphthyl) ethylamine prepared in Example 7 was charged into a reaction flask; by following the same operation steps as Example 8, 23.4 g of white crystalline agomelatine was obtained. The yield was 85% and the purity was 99.8%. The HPLC chromatogram was shown in Figure 3.

## Claims

1. A compound of formula I, 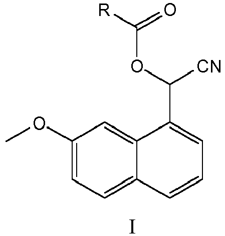 wherein R is methyl, ethyl or propyl.

2. A method for preparing the compound of formula I according to claim 1, wherein a compound (7-methoxyl-3,4-dihydro-2H-naphth-1-ylidene) acetonitrile of formula II reacts with organic acid to obtain the compound of formula I in the presence of aromatization reagent,

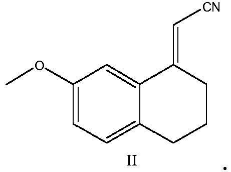

3. The preparation method according to claim 2, wherein the mole ratio of the aromatization reagent to the compound of formula II is 1-10:1.

4. The preparation method according to claim 2 or 3, wherein the aromatization reagent is 2,3-dichloro-5,6-dicyano-1,4-benzoquinone or tetrachlorobenzoquinone.

5. The preparation method according to any of claims 2-4, wherein the organic acid is selected from any one of acetic acid, propanoic acid and n-butyric acid.

6. A synthetic method of agomelatine, wherein the method comprises that the compound of formula I in an ammoniacal C₁-C₄ fatty alcohol is subjected to hydrogenation reduction under catalyzation by Raney nickel to prepare 2-(7-methoxyl-1-naphthyl) ethylamine of formula IV; 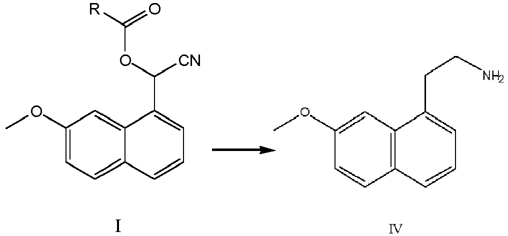 wherein R is methyl, ethyl or propyl.

7. The synthetic method according to claim 6, wherein the pressure for the hydrogenation reduction reaction is 0.1-2 Mpa.

8. The synthetic method according to claim 6 or 7, wherein the C₁-C₄ fatty alcohol is selected from methanol, ethanol, propanol, isopropanol, n-butanol, isobutanol or tertiary butanol.

9. The synthetic method according to any of claims 6-8, wherein the synthetic method also comprises that 7-methoxyl-1-tetralone of formula VIII, 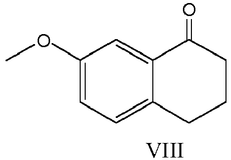 under catalyzation by sodium hydride, reacts with dimethyl cyanomethylphosphonate in an aprotic polar solvent to obtain (7-methoxyl-3, 4-dihydro-2H-naphth-1-ylidene) acetonitrile of formula II,

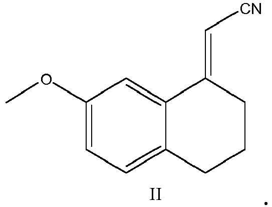

10. The synthetic method according to claim 9, wherein the aprotic polar solvent is N,N-dimethylformamide, N,N-dimethylacetamide, ethylene glycol dimethyl ether, dimethyl sulfoxide or sulfolane.

11. The synthetic method according to any of claims 6-10, wherein the synthetic method further comprises that 2-(7-methoxyl-1-naphthyl) ethylamine reacts with acetic anhydride to generate agomelatine which is then separated in solid form.

12. The synthetic method according to any of claims 6-11, comprising the following steps:
1) Charging ethylene glycol dimethyl ether and sodium hydride into a reaction vessel, controlling the temperature at 15±2 °C, adding the compound of formula (VIII), adding dimethyl cyanomethylphosphonate dropwise within 3-4 hours; controlling the temperature at 10 °C and stirring the mixture for 15 hours; cooling the mixture to a temperature below 5 °C, then slowly adding a small amount of water to complete the remaining sodium hydride; and then adding 10% hydrochloric acid solution dropwise to adjust the pH to 5-6; adding dichloromethane for extraction, and concentrating the organic layer under reduced pressure until dry; adding ethanol into the residue for dissolution by heating, and then cooling the resulting solution for crystallization, followed by filtration to give (7-methoxyl-3,4-dihydro-2H-naphth-1-ylidene) acetonitrile;
2) Charging tetrachlorobenzoquinone, glacial acetic acid and (7-methoxyl-3,4-dihydro-2H-naphth-1-ylidene) acetonitrile prepared in step 1 into a reaction vessel, followed by stirring; heating the mixture to refluxing, and allowing for reaction for 12 hours; cooling the reaction solution to room temperature, then distilling the reaction solution under reduced pressure; dissolving the residue by adding toluene followed by filtration; washing the filtrate with 5% sodium sulfite solution and then with water, followed by filtration, allowing for layering; concentrating the toluene layer under reduced pressure, dissolving the residue by adding ethanol followed by stirring, cooling the mixture for crystallization, followed by filtration to give 1-cyano-1-(7- methoxyl-1-naphthyl) methyl acetate;
3) Charging 1-cyano-1-(7-methoxyl-1-naphthyl) methyl acetate prepared in step 1, Raney nickel, ethanol and ammonia water into an autoclave which is then purged with nitrogen, introducing hydrogen gas; controlling the pressure at 0.3-0.8 Mpa and the temperature at 40-60 °C, allowing for reaction for 7 hours; cooling the reaction solution followed by filtration to recover Raney nickel; concentrating the filtrate under reduced pressure until dry, followed by washing by adding toluene and water, allowing for layering; and then washing the toluene layer by adding saturated brine, allowing for layering; concentrating the toluene layer under reduced pressure to obtain 1-(7-methoxyl-1-naphthyl) ethylamine;
4) Charging toluene, triethylamine and 2-(7-methoxyl-1-naphthyl) ethylamine prepared in step 3 into a reaction vessel, adding acetic anhydride dropwise slowly at a temperature below 30 °C; upon completion, allowing for reaction for 5 hours at 20-30 °C; after the completion of the reaction, adding water dropwise to hydrolyze the remaining acetic anhydride, followed by filtration; washing the filter cake with water to pH=7, washing the toluene layer with water to pH=7, and then pooling the filter cake and the toluene layer of the filtrate, followed by concentration under reduced pressure until dry; adding toluene into the residue, heating for refluxing until the solution turns clear; cooling the solution to 0-5 °C to allow for crystallization, followed by filtration; drying the filter cake to give agomelatine.

## Patentansprüche

1. Verbindung der Formel I, 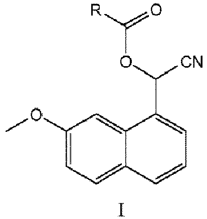 wobei R Methyl, Ethyl oder Propyl ist.

2. Verfahren zum Zubereiten der Verbindung der Formel I gemäß Anspruch 1, wobei eine Verbindung (7-Methoxyl-3,4-dihydro-2H-naphth-1-yliden)-Acetonitril der Formel II mit organischer Säure reagiert, um die Verbindung der Formel I in der Gegenwart von einem Aromatisierungsreagens zu erhalten,

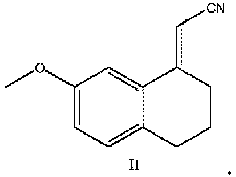

3. Zubereitungsverfahren gemäß Anspruch 2, wobei das Molverhältnis des Aromatisierungsreagens zu der Verbindung der Formel II 1 bis 10:1 ist.

4. Zubereitungsverfahren gemäß Anspruch 2 oder 3, wobei das Aromatisierungsreagens 2,3-Dichlor-5,6-dicyano-1,4-benzoquinon oder Tetrachlorbenzochinon ist.

5. Zubereitungsverfahren gemäß einem der Ansprüche 2 bis 4, wobei die organische Säure aus einer von Essigsäure, Propionsäure und n-Buttersäure ausgewählt wird.

6. Synthetisches Verfahren von Agomelatin, wobei das Verfahren umfasst, dass die Verbindung der Formel I in einem ammoniakalischen C₁-C₄-Fettalkohol einer Hydrierungsreduzierung unter Katalyse durch Raney-Nickel zum Zubereiten von 2-(7-Methoxyl-1-Naphthyl)-Ethylamin der Formel IV unterzogen wird; 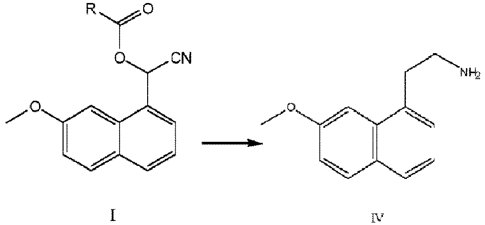 wobei R Methyl, Ethyl oder Propyl ist.

7. Synthetisches Verfahren gemäß Anspruch 6, wobei der Druck für die Hydrierungsreduzierungsreaktion 0,1 bis 2 Mpa ist.

8. Synthetisches Verfahren gemäß Anspruch 6 oder 7, wobei der C₁-C₄-Fettalkohol aus Methanol, Ethanol, Propanol, Isopropanol, n-Butanol, Isobutanol oder tertiärem Butanol ausgewählt wird.

9. Synthetisches Verfahren gemäß einem der Ansprüche 6 bis 8, wobei das synthetische Verfahren auch umfasst, dass 7-Methoxyl-1-tetralon der Formel VIII 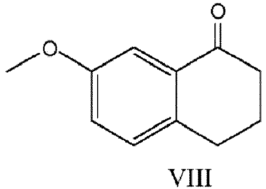 unter Katalyse durch Natriumhydrid mit Dimethyl-Cyanomethylphosphonat in einem aprotischen polaren Lösungsmittel reagiert, um (7-Methoxyl-3,4-dihydro-2H-naphth-1-yliden)-Acetonitril der Formel II zu erhalten.

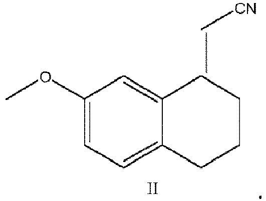

10. Synthetisches Verfahren gemäß Anspruch 9, wobei das aprotische polare Lösungsmittel N,N-Dimethylformamid, N,N-Dimethylacetamid, Ethylenglycoldimethylether, Dimethylsulfoxid oder Sulfolan ist.

11. Synthetisches Verfahren gemäß einem der Ansprüche 6 bis 10, wobei das synthetische Verfahren des Weiteren umfasst, dass 2-(7-Methoxyl-1-naphthyl)-Ethylamin mit Essigsäureanhydrid reagiert, um Agomelatin zu erzeugen, das danach in fester Form abgetrennt wird.

12. Synthetisches Verfahren gemäß einem der Ansprüche 6 bis 11, das die folgenden Schritte umfasst:
1) Beschicken eines Reaktionsgefäßes mit Ethylenglykol-Dimethylether und Natriumhydrid , Regeln der Temperatur auf 15±2 °C, Zugeben der Verbindung der Formel (VIII), tropfenweises Zugeben von Dimethyl-Cyanomethyplphosphonat innerhalb von 3 bis 4 Stunden; Regeln der Temperatur auf 10 °C und Rühren der Mischung für 15 Stunden; Kühlen der Mischung auf eine Temperatur unter 5 °C, danach langsames Zugeben einer kleinen Wassermenge, um das verbleibende Natriumhydrid zu vervollständigen; und danach tropfenweises Zugeben von 10 %iger Chlorwasserstoffsäurelösung, um den pH auf 5 bis 6 anzupassen; Zugeben von
Dichlormethan für eine Extraktion, und Konzentrieren der organischen Schicht unter vermindertem Druck, bis sie trocken ist; Zugeben von Ethanol zum Rückstand zur Auflösung durch Erhitzen und danach Kühlen der entstandenen Lösung zur Kristallisation, gefolgt von Filtration, um (7-Methoxyl-3,4-dihydro-2H-naphth-1-yliden)-Acetonitril zu ergeben;
2) Beschicken eines Reaktionsgefäßes mit Tetrachlorbenzochinon, Eisessig und (7-Methoxyl-3,4-dihydro-2H-naphth-1-yliden)-Acetonitril, das in Schritt 1 vorbereitet wurde, gefolgt von Rühren; Erhitzen der Mischung bis zum Reflux und Zulassen der Reaktion für 12 Stunden, Kühlen der Reaktionslösung auf Raumtemperatur, danach Destillieren der Reaktionslösung unter vermindertem Druck;
Auflösen des Rückstands durch Zugeben von Toluol, gefolgt von Filtration; Waschen des Filtrats mit 5 %iger Natriumsulfitlösung und danach mit Wasser, gefolgt von Filtration, Zulassen des Schichtbildens; Konzentrieren der Toluolschicht unter vermindertem Druck, Auflösen des Rückstands durch Zugeben von Ethanol, gefolgt von Rühren, Kühlen der Mischung zur Kristallisation, gefolgt von Filtration, um 1-Cyano-1-(7-methoxyl-1-naphthyl)-Methylacetat zu ergeben;
3) Beschicken eines Autoklaven mit 1-Cyano-1-(7-methoxyl-1-naphthyl)-Methylacetat, das in Schritt 1 zubereitet wurde, Raney-Nickel, Ethanol und Ammoniakwasser, der danach mit Stickstoff unter Einleiten von Knallgas bespült wird; Regeln des Drucks auf 0,3 bis 0,8 Mpa und der Temperatur auf 40 bis 60 °C, Zulassen der Reaktion für 7 Stunden; Kühlen der Reaktionslösung, gefolgt von Filtration, um Raney-Nickel zurückzugewinnen; Konzentrieren des Filtrats unter vermindertem Druck, bis es trocken ist, gefolgt von Waschen durch Zugeben von Toluol und Wasser, Zulassen des Schichtbildens; und danach Waschen der Toluolschicht durch Zugeben von gesättigter Sole, Zulassen des Schichtbildens; Konzentrieren der Toluolschicht unter vermindertem Druck, um 1-(7-Methoxyl-1-naphthyl)-Ethylamin zu erhalten;
4) Beschicken eines Reaktionsgefäßes mit Toluol, Triethylamin und 2-(7-Methoxyl-1-naphthyl)-Ethylamin, das in Schritt 3 zubereitet wurde, , langsames, tropfenweises Zugeben von Essigsäureanhydrid bei einer Temperatur unter 30 °C; nach Abschluss Zulassen der Reaktion für 5 Stunden bei 20 bis 30 °C; nach Abschluss der Reaktion tropfenweises Zugeben von Wasser, um das verbleibende Essigsäureanhydrid zu hydrolysieren, gefolgt von Filtration; Waschen des Filterkuchens mit Wasser bis pH=7, Waschender Toluolschicht mit Wasser bis pH=7, und danach Zusammengeben des Filterkuchens und der Toluolschicht des Filtrats, gefolgt von Konzentration unter vermindertem Druck, bis es trocken ist. Zugeben von Toluol zu dem Rückstand, Erhitzen unter Rückfluss, bis die Lösung klar wird; Kühlen der Lösung auf 0 bis 5 °C, um Kristallisation zuzulassen, gefolgt von Filtration; Trocknen des Filterkuchens, um Agomelatin zu ergeben.

## Revendications

1. Un composé de formule I, 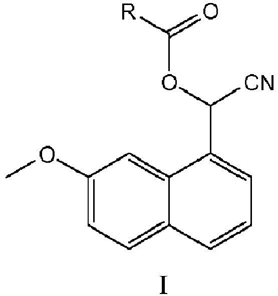 dans laquelle R est un méthyle, un éthyle ou un propyle.

2. Un procédé de préparation du composé de formule I selon la revendication 1, dans lequel un composé (7-méthoxyl-3,4-dihydro-2H-napht-1-ylidène) acétonitrile de formule II réagit avec de l'acide organique pour obtenir le composé de formule I en présence d'un réactif d'aromatisation,

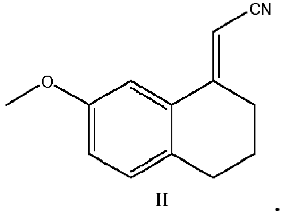

3. Le procédé de préparation selon la revendication 2, dans lequel le rapport molaire du réactif d'aromatisation au composé de formule II est de 1 à 10:1.

4. Le procédé de préparation selon la revendication 2 ou 3, dans lequel le réactif d'aromatisation est le 2,3-dichloro-5,6-dicyano-1,4-benzoquinone ou le tétrachlorobenzoquinone.

5. Le procédé de préparation selon l'une quelconque des revendications 2 à 4, dans lequel l'acide organique est choisi parmi n'importe lequel de l'acide acétique, l'acide propanoïque et l'acide n-butyrique.

6. Un procédé de synthèse de l'agomélatine, dans lequel le procédé comprend le fait que le composé de formule I dans un alcool gras en C₁-C₄ ammoniacal est soumis à une réduction d'hydrogénation sous catalyse par du nickel de Raney pour préparer du 2-(7-méthoxyl-1-naphtyl) éthylamine de formule IV ; 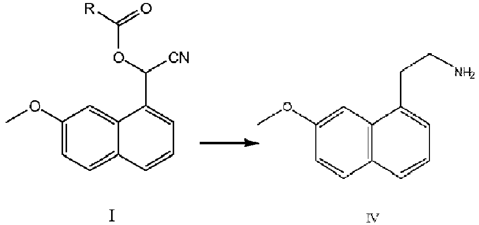 dans laquelle R est un méthyle, un éthyle ou un propyle.

7. Le procédé de synthèse selon la revendication 6, dans lequel la pression pour la réaction de réduction d'hydrogénation est de 0,1 à 2 Mpa.

8. Le procédé de synthèse selon la revendication 6 ou 7, dans lequel l'alcool gras en C₁-C₄ est choisi parmi le méthanol, l'éthanol, le propanol, l'isopropanol, le n-butanol, l'isobutanol ou le butanol tertiaire.

9. Le procédé de synthèse selon l'une quelconque des revendications 6 à 8, dans lequel le procédé de synthèse comprend également le fait que la 7-méthoxyl-1-tétralone de formule VIII, 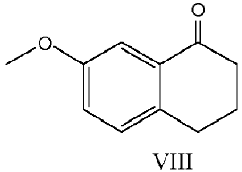 sous catalyse par l'hydrure de sodium, réagit avec le diméthyl cyanométhylphosphonate dans un solvant polaire aprotique pour obtenir du (7-méthoxyl-3, 4-dihydro-2 H-napht-1-ylidène) acétonitrile de formule II,

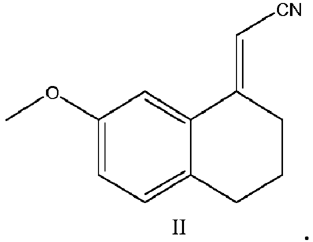

10. Le procédé de synthèse selon la revendication 9, dans lequel le solvant polaire aprotique est le N,N-diméthylformamide, le N,N-diméthylacétamide, l'éther diméthylique d'éthylène glycol, le diméthylsulfoxyde ou le sulfolane.

11. Le procédé de synthèse selon l'une quelconque des revendications 6 à 10, dans lequel le procédé de synthèse comprend en outre le fait que le 2-(7-méthoxyl-1-naphtyl) éthylamine réagit avec l'anhydride acétique pour générer de l'agomélatine qui est ensuite séparée sous forme solide.

12. Le procédé de synthèse selon l'une quelconque des revendications 6 à 11 comprenant les étapes suivantes :
1) le chargement de l'éther diméthylique d'éthylène glycol et de l'hydrure de sodium dans un récipient de réaction, la régulation de la température à 15±2 °C, l'ajout du composé de formule (VIII), l'ajout de diméthyl cyanométhylphosphonate goutte à goutte en 3 à 4 heures ; la régulation de la température à 10 °C et l'agitation du mélange pendant 15 heures; le refroidissement du mélange à une température inférieure à 5 °C, puis l'ajout lentement d'une petite quantité d'eau pour achever l'hydrure de sodium restant ; et l'ajout ensuite d'une solution d'acide chlorhydrique à 10 % goutte à goutte pour ajuster le pH de 5 à 6 ; l'ajout de dichlorométhane pour l'extraction, et la concentration de la couche organique sous une pression réduite jusqu'à ce qu'elle soit sèche ; l'ajout d'éthanol dans le résidu pour une dissolution par chauffage, puis le refroidissement de la solution résultante pour une cristallisation, suivie d'une filtration pour donner du (7-méthoxyl-3,4-dihydro-2H-napht-1-ylidène) acétonitrile ;
2) le chargement de tétrachlorobenzoquinone, d'acide acétique glacial et de (7-méthoxyl-3,4-dihydro-2H-napht-1-ylidène) acétonitrile préparé à l'étape 1 dans un récipient de réaction, suivi d'une agitation ; le chauffage du mélange à reflux, pour permettre une réaction pendant 12 heures ; le refroidissement de la solution de réaction à la température ambiante, puis la distillation de la solution de réaction sous une pression réduite ; la dissolution du résidu en ajoutant du toluène suivi d'une filtration ; le lavage du filtrat avec une solution de sulfite de sodium à 5 % puis avec de l'eau, suivi d'une filtration, permettant la stratification ; la concentration de la couche de toluène sous une pression réduite, la dissolution du résidu en ajoutant de l'éthanol suivi d'une agitation, le refroidissement du mélange pur une cristallisation, suivie d'une filtration pour donner de l'acétate de 1-cyano-1-(7-méthoxyl-1-naphtyl) méthyle ;
3) le chargement d'acétate de 1-cyano-1-(7-méthoxyl-1-naphtyl) méthyle préparé à l'étape 2, du nickel de Raney, de l'éthanol et de l'eau ammoniacale dans un autoclave qui est ensuite purgé avec de l'azote, l'introduction d'hydrogène gazeux; la régulation de la pression de 0,3 à 0,8 Mpa et de la température de 40 à 60 °C, permettant une réaction pendant 7 heures; le refroidissement de la solution de réaction suivie d'une filtration pour récupérer le nickel de Raney ; la concentration du filtrat sous une pression réduite jusqu'à ce qu'il soit sec, suivie d'un lavage par ajout de toluène et d'eau, ce qui permet une stratification ; puis le lavage de la couche de toluène en ajoutant de la saumure saturée, ce qui permet une stratification ; la concentration de la couche de toluène sous pression réduite pour obtenir de la 1-(7-méthoxyl-1-naphtyl) éthylamine ;
4) Le chargement de toluène, de triéthylamine et de 2-(7-méthoxyl-1-naphtyl) éthylamine préparée à l'étape 3 dans un récipient de réaction, l'ajout d'anhydride acétique goutte à goutte lentement à une température inférieure à 30 °C ; lors de l'achèvement, permettre une réaction pendant 5 heures de 20 à 30 °C ; après la fin de la réaction, l'ajout d'eau goutte à goutte pour hydrolyser l'anhydride acétique restant, suivie d'une filtration ; le lavage du gâteau de filtration avec de l'eau à un pH=7, le lavage de la couche de toluène avec de l'eau à un pH=7, puis la mise en commun du gâteau de filtration et de la couche de toluène du filtrat, suivie d'une concentration sous pression réduite jusqu'à ce qu'elle soit sèche ; l'ajout de toluène dans le résidu, le chauffage à reflux jusqu'à ce que la solution soit claire ; le refroidissement de la solution de 0 à 5 °C pour permettre une cristallisation, suivie d'une filtration ; le séchage du gâteau de filtration pour donner de l'agomélatine.
